(19) Europäisches Patentamt / European Patent Office / Office européen des brevets

(11) **EP 4 640 813 A1**

(12) **EUROPEAN PATENT APPLICATION**
published in accordance with Art. 153(4) EPC

(43) Date of publication:
**29.10.2025 Bulletin 2025/44**

(21) Application number: **23906379.5**

(22) Date of filing: **31.08.2023**

(51) International Patent Classification (IPC):
*C12M 1/00* (2006.01)   *C12M 3/04* (2006.01)
*C12N 5/071* (2010.01)

(52) Cooperative Patent Classification (CPC):
**C12M 1/00; C12M 3/04; C12N 5/06**

(86) International application number:
**PCT/JP2023/032030**

(87) International publication number:
**WO 2024/135002 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2022 JP 2022204758**
**21.12.2022 JP 2022204761**

(71) Applicant: **Mitsui Chemicals, Inc.**
**Tokyo 104-0028 (JP)**

(72) Inventors:
• **SAKAJIRI, Aya**
**Tokyo 104-0028 (JP)**
• **ESASHIKA, Katsuhiro**
**Tokyo 104-0028 (JP)**

(74) Representative: **J A Kemp LLP**
**80 Turnmill Street**
**London EC1M 5QU (GB)**

(54) **CULTURE CONTAINER, METHOD FOR PRODUCING CULTURE CONTAINER, AND METHOD FOR CULTURING SPHEROID**

(57) Provided is a culture plate having a plurality of accommodating parts for culturing cells, said culture plate comprising a base material which has a plurality of through holes and a gas-permeable sheet which has a flat part that is adhered to a lower surface of the base material and a plurality of recessed parts that are positioned below the through holes and that form the accommodating parts by closing the through holes.

FIG. 2

## Description

The present invention relates to a culture plate, a method for manufacturing a culture plate, and a method for culturing a spheroid.

Background Art

Conventionally, as disclosed in PTL 1, cells have been cultured using a culture plate that includes an accommodation part for culturing cells.

Citation List

Patent Literature

PTL 1
Japanese Patent Application Laid-Open No. 2022-149254

Summary of Invention

Technical Problem

In recent years, culture plates with high culture functions have been in demand.
An object of the present invention is to provide a culture plate with a high culture function, a method for manufacturing a culture plate, and a method for culturing a spheroid.

Solution to Problem

A culture plate a culture plate according to the present invention includes: a plurality of accommodation parts for culturing cells; a base material including a plurality of through-holes; and a gas-permeable sheet, the gas-permeable sheet including: a flat surface part attached to a lower surface of the base material, and a plurality of recesses disposed below the through-holes so as to close the through-holes and form the accommodation parts.
A method for manufacturing a culture plate according to the present invention is a method including: providing a base material including a plurality of through-holes; obtaining a gas-permeable sheet by deforming a planar sheet and forming a plurality of recesses at positions corresponding to the through-holes; and bonding the gas-permeable sheet to the base material in a state in which the plurality of recesses are disposed at positions of the plurality of through-holes.
A method for culturing a spheroid according to the present invention is implemented by using the above-described culture plate.

Advantageous Effects of Invention

According to the present invention, it is possible to provide a culture plate with a high culture function, a method for manufacturing a culture plate, and a method for culturing a spheroid.

Brief Description of Drawings

FIG. 1 is a perspective view of a culture plate according to an embodiment;
FIG. 2 is a partial cross-sectional view of the culture plate;
FIG. 3A is a cross-sectional schematic view illustrating a modification example of a gas-permeable sheet;
FIG. 3B is a cross-sectional schematic view illustrating a modification example of the gas-permeable sheet;
FIG. 3C is a cross-sectional schematic view illustrating a modification example of the gas-permeable sheet;
FIG. 3D is a cross-sectional schematic view illustrating a modification example of the gas-permeable sheet;
FIG. 3E is a cross-sectional schematic view illustrating a modification example of the gas-permeable sheet;
FIG. 3F is a cross-sectional schematic view illustrating a modification example of the gas-permeable sheet;
FIG. 3G is a cross-sectional schematic view illustrating a modification example of the gas-permeable sheet;
FIG. 4 is a flowchart illustrating a step of manufacturing a culture plate;
FIG. 5 is a schematic perspective view of a sheet material used in the method for manufacturing a culture plate;
FIG. 6 is a schematic perspective view of an intermediate generation member manufactured in the method for manufacturing a culture plate;
FIG. 7 is a schematic perspective view of a jig used in the method for manufacturing a culture plate;
FIG. 8 is a cross-sectional schematic view illustrating a state in which a jig is assembled to the intermediate generation member;
FIG. 9 is a schematic view for describing a step of manufacturing of a culture plate;
FIG. 10 is a schematic diagram for describing a step of manufacturing a culture plate;
FIG. 11 is a schematic diagram for describing a step of manufacturing a culture plate;
FIG. 12 is a schematic view illustrating a modification example of the manufacturing apparatus; and
FIG. 13 is a schematic view illustrating a modification example of the manufacturing apparatus.

Description of Embodiments

Hereinafter, a culture plate, a method for manufacturing a culture plate, and a method for culturing a spheroid according to the present invention will be described in detail with reference to the drawings. Note that the culture plate described below is an example of a culture plate according to the present invention, and the present invention is not limited to the embodiments described later.

Embodiment

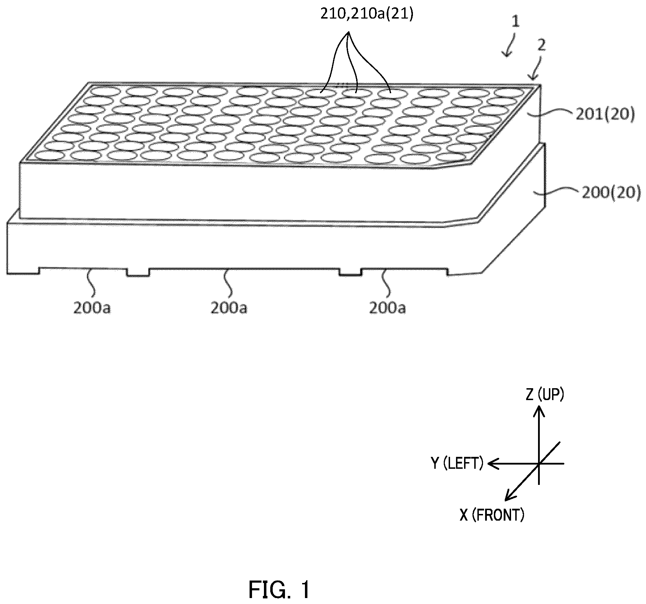

With reference to FIGS. 1 to 11, a culture plate, a method for manufacturing a culture plate, and a method for culturing a spheroid according to an embodiment of the present invention will be described.

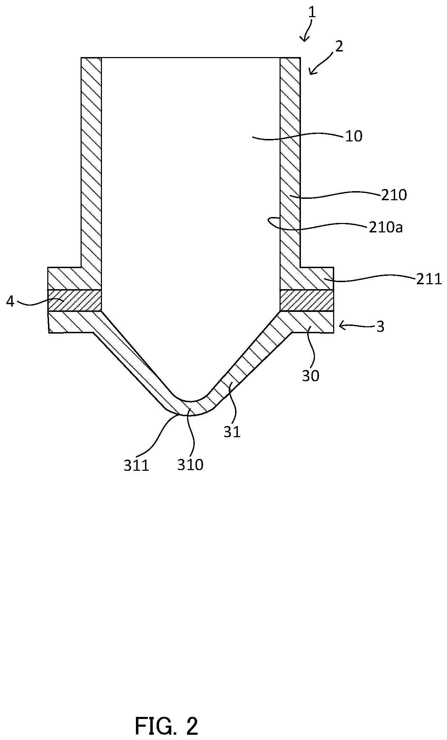

FIG. 1 is a perspective view of culture plate 1 according to an embodiment of the present invention. FIG. 2 is a partial cross-sectional view of culture plate 1.

Culture plate 1 is used, for example, for culturing cells derived from a human. Culture plate 1 as described above includes a plurality of accommodation parts 10 for culturing cells. Culture plate 1 is accommodated in a culture space of a culture apparatus (for example, an incubator) in a state in which a cell to be cultured (hereinafter referred to as "target cell") is accommodated in accommodation part 10.

Culture plate 1 of the present embodiment can be suitably used for culturing spheroids (cell aggregates) of target cells. Note that culture plate 1 does not need to be used in a state of being accommodated in the culture apparatus. Culture plate 1 may be used in various situations according to the target cell.

Hereinafter, a specific configuration of culture plate 1 will be described. Subsequently, a method for manufacturing culture plate 1 will be described.

Culture plate 1 includes main body part 2, gas-permeable sheet 3, and adhesive layer 4.

Note that in describing the structure of culture plate 1, the orthogonal coordinate system (X, Y, Z) illustrated in FIG. 1 may be used. The X-direction corresponds to the front-rear direction of culture plate 1. The X-direction + side corresponds to the front side of culture plate 1. The X-direction - side corresponds to the rear side of culture plate 1. The Y-direction corresponds to the left-right direction of culture plate 1. The Y-direction + side corresponds to the left side of culture plate 1. The Y-direction - side corresponds to the right side of culture plate 1. The Z-direction corresponds to the up-down direction of culture plate 1. The Z-direction + side corresponds to the upper side of culture plate 1. The Z-direction - side corresponds to the lower side of culture plate 1. Note that the left side, right side, front side, and rear side of culture plate 1 vary depending on the direction in which culture plate 1 is viewed, and are not limited to the left side, right side, front side, and rear side of culture plate 1 illustrated in FIG. 1.

Main body part 2 corresponds to an example of a base material, and includes frame part 20 and main body-side accommodation part 21. Main body part 2 is, for example, made of a synthetic resin and is an integrally molded article manufactured by injection molding. The synthetic resin that constitutes main body part 2 includes, for example, polystyrene and polyolefin. The polyolefin may be a cyclic olefin (co)polymer, a 4-methyl-1-pentene (co)polymer, polypropylene, or polyethylene. From the viewpoint of gas permeability, a 4-methyl-1-pentene-based (co)polymer is preferred. Further, from the viewpoint of heat resistance, polystyrene, polypropylene, or a cyclic olefin (co)polymer is preferable. Note that the synthetic resin constituting the main body part (that is, the base material) may be a thermoplastic resin. In this case, the main body part (that is, the base material) may be constituted of a thermoplastic resin having a glass transition temperature of -50°C to 180°C.

The glass transition temperature of the thermoplastic resin constituting main body part 2 is preferably higher by 40°C or more than the glass transition temperature of the thermoplastic resin constituting gas-permeable sheet 3 described later.

The glass transition temperature is the peak temperature of the loss tangent $\tan\delta$ determined by the following Equation (1). E' in the following Equation (1) is a storage elastic modulus. Further, E" in the following Equation (1) is the loss elastic modulus.
[1]

$$\tan\delta = E'' / E' \ldots (\text{Equation 1})$$

The storage elastic modulus (E') and the loss elastic modulus (E") were measured using a measurement apparatus (DVA-225 manufactured by IT Keisoku Seigyo Co., Ltd.). The conditions for the measurement test are as follows.

Measurement mode of the measurement apparatus: Tension mode
Size of test piece: 45 mm × 10 mm
Heating rate: 3°C/min
Frequency of sine wave: 1 Hz
Measurement temperature range: -150°C to 200°C

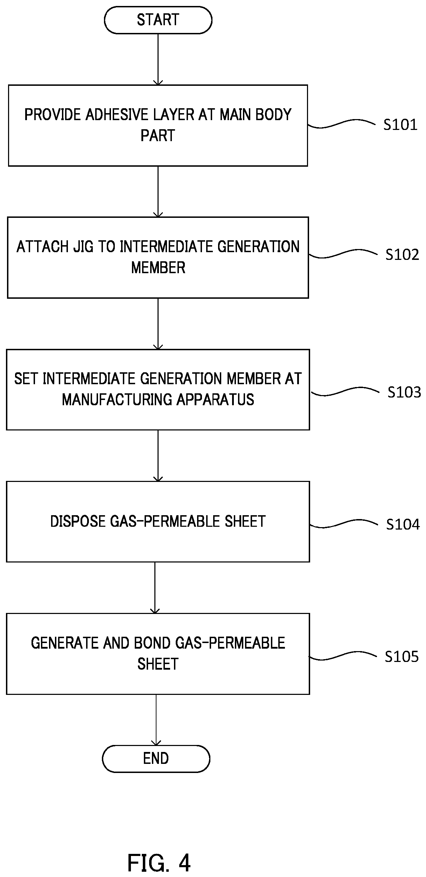

The instantaneous value of the Shore D hardness of main body part 2 is, for example, 60 to 90. The instantaneous value of the Shore D hardness is a value obtained by reading the scale immediately after bringing the indenter into contact with a part of main body part 2 using a durometer (type D) (in accordance with ASTM D2240). When the instantaneous value of the Shore D hardness is in a range of 60 to 90, the gas-permeable sheet can be attached to main body part 2 with a good appearance in the step of attaching the gas-permeable sheet (the step is performed in step S105 in the flowchart illustrated in FIG. 4 described later).

Frame part 20 is constituted by a rectangular frame member. Frame part 20 constitutes the outer shape of culture plate 1.

Frame part 20 includes lower frame part 200 and upper frame part 201.

Lower frame part 200 is a rectangular frame member and constitutes the lower-side half of frame part 20. Lower frame part 200 corresponds to an example of the lower peripheral wall part.

Lower frame part 200 is a part that is placed on a placing part provided in, for example, a culture apparatus (not illustrated) in a use state of culture plate 1. Lower frame part 200 has a height such that the deepest part (in other words, the lower end part) of recess 31 of gas-permeable sheet 3 described later does not come into contact with the placing surface of the placing part.

Lower frame part 200 is provided to surround the side of the space, which is present on the lower side of main body-side accommodation part 21 described later, over the entire circumference. The space present on the lower side of main body-side accommodation part 21 is also a space present on the lower side of gas-permeable sheet 3 described later (hereinafter referred to as "lower space of gas-permeable sheet 3").

Lower frame part 200 may include notch part 200a at a plurality of locations of the lower end part. In the use state of culture plate 1, notch part 200a communicates a space on the inner peripheral surface side of lower side frame part 200 (that is, a space below gas-permeable sheet 3) with a space on the outer peripheral surface side of lower frame part 200 (that is, an external space).

Thus, in the use state of culture plate 1, air from the external space is supplied to the lower region of gas-permeable sheet 3 via notch part 200a.

As described above, the configuration in which lower frame part 200 includes notch part 200a contributes to the improvement in the oxygen supply property to the lower region of gas-permeable sheet 3. Further, the oxygen supplied to the lower region of gas-permeable sheet 3 is supplied to the target cells accommodated in accommodation part 10 of culture plate 1 through gas-permeable sheet 3, thereby promoting the culture of the target cells. Note that the position and/or the number of the notch part is not limited to the position and/or the number of the notch part 200a described above. Further, the notch part may be omitted.

Upper frame part 201 is a rectangular frame member and constitutes the upper half of frame part 20. Upper frame part 201 is provided on the upper side of lower frame part 200. Upper frame part 201 corresponds to an example of the upper peripheral wall part.

Upper frame part 201 is provided to surround main body-side accommodation part 21 over the entire circumference.

The upper end part of upper frame part 201 has a shape that fits with the lower end part of lower frame part 200 of another culture plate 1. That is, culture plate 1 is configured to be capable of being disposed by stacking a plurality of culture plate 1 vertically.

By fitting upper frame part 201 of culture plate 1 disposed on the lower side and lower frame part 200 of culture plate 1 disposed on the upper side, culture plate 1 disposed on the lower side and culture plate 1 disposed on the upper side can be stacked without rattling.

Main body-side accommodation part 21 is provided in a space surrounded by frame part 20. Main body-side accommodation part 21 is provided integrally with frame part 20.

Main body-side accommodation part 21 includes a plurality of cylindrical parts 210 and bottom plate part 211.

Cylindrical part 210 has a cylindrical shape that is open in the up-down direction. In the present embodiment, the shape of the opening part of cylindrical part 210 (in other words, the outer shape in plan view) is circular. Cylindrical part 210 is provided side by side in the left-right direction and the front-rear direction. The number of cylindrical parts 210 matches the number of accommodation parts 10 for culturing cells. The number of cylindrical parts 210 may be, for example, 6, 24, 96, or 384. Naturally, the number of cylindrical parts 210 may be other than 6, 24, 96, and 384.

Adjacent cylindrical parts 210 are connected to each other by a connecting part (not illustrated). Further, left end cylindrical part 210, right end cylindrical part 210, front end cylindrical part 210, and rear end cylindrical part 210 are connected to the inner peripheral surface of frame part 20 via a connecting part (not illustrated).

A space defined by the inner peripheral surface of cylindrical part 210 (hereinafter referred to as "inner space of cylindrical part 210") corresponds to an example of a through-hole in a base material. The inner space of cylindrical part 210, together with recess 31 of gas-permeable sheet 3 described later, constitutes accommodation part 10 for culturing cells.

As illustrated in FIG. 2, bottom plate part 211 is a plate-like member that connects the lower end parts of cylindrical part 210 to each other. The outer edge part of bottom plate part 211 is connected to the inner peripheral surface of frame part 20 over the entire circumference. Note that the lower surface of bottom plate part 211 is also the lower surface of main body-side accommodation part 21. Further, the lower surface of bottom plate part 211 is also the lower surface of main body part 2. Thus, the lower surface of bottom plate part 211 corresponds to an example of the lower surface of the base material.

Next, gas-permeable sheet 3 will be described with reference to FIG. 2. FIG. 2 is a partial cross-sectional view of culture plate 1. FIG. 2 is a cross-sectional view of a part of culture plate 1 corresponding to one cylindrical part 210.

Gas-permeable sheet 3 is bonded to the lower surface of main body part 2 via adhesive layer 4. Specifically, gas-permeable sheet 3 is bonded to the lower surface of bottom plate part 211 in main body-side accommodation part 21 via adhesive layer 4.

Gas-permeable sheet 3 is a sheet member having an oxygen permeability coefficient of 200 to 60,000 $cm^3 \times mm/(m^2 \times 24 \, hr \times atm)$. Gas-permeable sheet 3 is, for example, a sheet member including a polymer having a structural unit derived from 4-methyl-1-pentene. Note that the material of the gas-permeable sheet is not limited to a polymer having a structural unit derived from 4-methyl-1-pentene. The gas-permeable sheet may be made of various thermoplastic resins having a desired gas permeability (oxygen permeability coefficient). In this case, the gas-permeable sheet may be constituted of a thermoplastic resin having a glass transition temperature of -150°C to 80°C. Further, the thermosetting resin that constitutes the gas-permeable sheet may be different from the thermoplastic resin that constitutes the main body part (that is, the base material). Note that the method for determining the glass transition temperature of the material constituting gas-permeable sheet 3 is the same as the method for determining the glass transition temperature of the material constituting main body part 2.

In a case where gas-permeable sheet 3 is constituted by a 4-methyl-1-pentene-based (co)polymer, it is preferable that main body part 2 is also constituted by a 4-methyl-1-pentene-based (co)polymer. Further, in a case where gas-permeable sheet 3 is constituted by a 4-methyl-1-pentene-based (co)polymer, main body part 2 is preferably made of polystyrene.

Gas-permeable sheet 3 includes flat surface part 30 and a plurality of recesses 31.

Flat surface part 30 is a part of gas-permeable sheet 3 that is bonded to the lower surface of bottom plate part 211 in main body-side accommodation part 21. The thickness of flat surface part 30 is equal or substantially equal over the entire surface. The average thickness of flat surface part 30 is $T_2$.

Recess 31 is disposed below the inner space of cylindrical part 210 (that is, the through-hole of the base material) in gas-permeable sheet 3. Recess 31 has a substantially conical shape that is convex toward the lower side. Bottom part 310 (that is, the lower end part) of recess 31 is spherical.

Further, recess 31 includes curved surface part 311 (see FIG. 2) having a curved surface shape on the bottom surface. Curvature radius R1 of curved surface part 311 is, for example, $0.5 \, mm \leq R1 \leq 2 \, mm$.

Hereinafter, the inner space of cylindrical part 210 will be simply referred to as through-hole 210a. Recess 31 closes the lower side opening part of through-hole 210a. Through-hole 210a is open in the up-down direction.

Recess 31, together with through-hole 210a, constitutes accommodation part 10 of culture plate 1. The region constituted by recess 31 in accommodation part 10 is referred to as the lower side region of accommodation part 10. Recess 31 has a circular outer shape (outer edge part) in plan view. Note that through-hole 210a also has a circular outer shape (outer edge part) in plan view.

Note that the plan view means viewing culture plate 1 from above (Z direction + side) of culture plate 1. Hereinafter, unless otherwise specified, the outer shape of recess 31 refers to the outer shape of recess 31 in plan view. Further, the outer shape of through-hole 210a means the outer shape of through-hole 210a in plan view.

In plan view, the outer shape of recess 31 and the outer shape of through-hole 210a match or substantially match. In other words, the diameter of the outer shape of recess 31 and the diameter of the outer shape of through-hole 210a are equal to or substantially equal to each other. Specifically, the outer shape of through-hole 210a (in other words, the outer shape of the opening part of cylindrical part 210) may be circular, triangular, quadrangular, pentagonal, hexagonal, or any other polygonal shape.

The outer shape of through-hole 210a is preferably circular or rectangular. From the viewpoint of improving the adhesion strength between gas-permeable sheet 3 and the periphery of through-hole 210a, the outer shape of through-hole 210a is preferably circular because a circular outer shape of through-hole 210a can achieve more uniform bonding at the adhesion part between the periphery of through-hole 210a and gas-permeable sheet 3.

Further, from the viewpoint of increasing the occupied volume of through-hole 210a (in other words, the volume of accommodation part 10), the outer shape of through-hole 210a is preferably a quadrilateral. For example, in a case where the number of through-holes 210a is large (for example, 384), the volume of accommodation part 10 may be reduced if the outer shape of through-hole 210a is circular. In this case, when the outer shape of through-hole 210a is a polygon such as a quadrilateral, the volume of accommodation part 10 is more easily increased than when the outer shape of through-hole 210a is circular.

Thickness $T_1$ of recess 31 is smaller than thickness $T_2$ of flat surface part 30 ($T_1 < T_2$). Further, thickness $T_{10}$ of bottom part 310 in recess 31 is smaller than the thickness of the peripheral part of bottom part 310. That is, the thickness of bottom part 310 is the thinnest in recess 31. In other words, the thickness of recess 31 decreases toward bottom part 310. Note that the thickness of the predetermined part of recess 31 means the thickness in the direction of the perpendicular line at the predetermined part.

Further, maximum thickness $T_{1max}$ of bottom part 310 and average thickness $T_2$ of flat surface part 30 satisfy a relationship of $0.5T_2 \leq T_{1max} \leq 0.9T_2$. Further, average thickness $T_2$ of flat surface part 30 and thickness $T_{1mid}$ of the central part of recess 31 in the up-down direction satisfy a relationship of $0.65T_2 \leq T_{1mid} \leq 0.98T_2$.

As described above, recess 31 is configured such that the thickness of bottom part 310 is the thinnest. For this reason, recess 31 has the highest oxygen permeability at bottom part 310. During the use of culture plate 1, the target cells accommodated in accommodation part 10 gather at bottom part 310 having the highest oxygen permeability in recess 31. In this manner, the configuration of recess 31 in the present embodiment contributes to promoting the culture of target cells.

Note that recess 31 as described above is formed by deforming a flat gas-permeable sheet. A method for forming recess 31 will be described later.

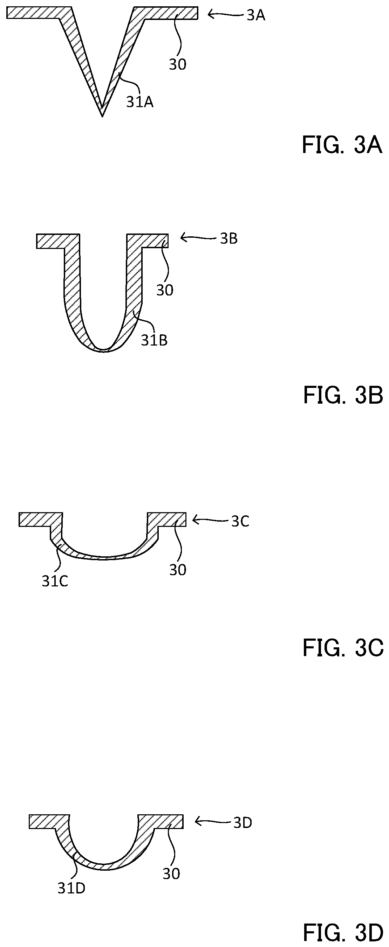

Note that the configuration of the gas-permeable sheet is not limited to the configuration of gas-permeable sheet 3 described above. FIGS. 3A to 3G are views illustrating a modification example of the gas-permeable sheet. Gas-permeable sheets 3A to 3G illustrated in FIGS. 3A to 3G differ from recess 31 in the above-described embodiment in the configuration of the recess.

Configurations of gas-permeable sheets 3A to 3G according to modification examples will be briefly described below. Note that the same configuration of gas-permeable sheets 3A to 3G as that of gas-permeable sheet 3 in the above-described embodiment will be omitted as appropriate.

First, gas-permeable sheet 3A according to modification example 1 illustrated in FIG. 3A includes recess 31A having a substantially V-shaped cross-sectional shape. The depth of recess 31A is greater than the depth of recess 31 of the above-described Embodiment 1.

Recess 31A is disposed below through-hole 210a constituted by the inner space of cylindrical part 210 (see FIG. 2). Recess 31A closes the lower side opening part of through-hole 210a (see FIG. 2). In the present example, bottom part 310 (that is, the lower end part) of recess 31A is pointed.

Recess 31A, together with through-hole 210a, constitutes accommodation part 10 of culture plate 1. Recess 31 has a circular outer shape (outer edge part) in plan view.

The diameter of the outer shape of recess 31A and the diameter of the outer shape of through-hole 210a are equal to or substantially equal to each other. Note that the thickness relationship between recess 31A and flat surface part 30 is the same as the thickness relationship between recess 31 and flat surface part 30 in the above-described embodiment.

Next, gas-permeable sheet 3B according to modification example 2 illustrated in FIG. 3B includes recess 31B having a substantially U-shaped cross-sectional shape. The depth of recess 31B is greater than the depth of recess 31 of the above-described Embodiment 1. Recess 31B is disposed below through-hole 210a constituted by the inner space of cylindrical part 210 (see FIG. 2).

Recess 31B is constituted by a cylindrical part that is convex toward the lower side and a hemispherical part that is provided at the lower end of the cylindrical part. Recess 31B closes the lower side opening part of through-hole 210a (see FIG. 2). Note that the structure of the recess in the gas-permeable sheet is not limited to the structure of recess 31B. For example, the outer shape of the recess in the gas-permeable sheet may be a shape corresponding to the outer shape of cylindrical part 210 (that is, through-hole 210a) in main body part 2 described above. Specifically, the outer shape of the recess in the gas-permeable sheet may be circular, triangular, quadrangular, pentagonal, hexagonal, or any other polygonal shape.

Recess 31B is constituted by a cylindrical part that is convex toward the lower side and a hemispherical part that is provided at the lower end of the cylindrical part.

Recess 31B, together with through-hole 210a, constitutes accommodation part 10 of culture plate 1. Recess 31 has a circular outer shape (outer edge part) in plan view.

The diameter of the outer shape of recess 31B and the diameter of the outer shape of through-hole 210a are equal to or substantially equal to each other. Note that the thickness relationship between recess 31B and flat surface part 30 is the same as the thickness relationship between recess 31 and flat surface part 30 in the above-described embodiment.

Next, gas-permeable sheet 3C according to modification example 3 illustrated in FIG. 3C includes recess 31B having a substantially U-shaped cross-sectional shape. The depth of recess 31B is shallower than the depth of recess 31B in the above-described modification example 2.

Recess 31C is constituted by a cylindrical part that is convex toward the lower side and a hemispherical part that is provided at the lower end of the cylindrical part. The length of the cylindrical part of recess 31C is shorter than the length of the cylindrical part of recess 31B in modification example 2. Further, the inner diameter of the cylindrical part of recess 31C is larger than the inner diameter of recess 31B in modification example 2. The other configurations of recess 31C are substantially the same as the configurations of recess 31B in modification example 2.

Next, gas-permeable sheet 3D according to modification example 4 illustrated in FIG. 3D includes recess 31D having a substantially semicircular cross-sectional shape. Recess 31D is disposed below through-hole 210a constituted by the inner space of cylindrical part 210 (see FIG. 2).

Recess 31D is constituted by a hemispherical part that is convex toward the lower side. Recess 31D closes the lower side opening part of through-hole 210a (see FIG. 2).

Recess 31D, together with through-hole 210a, constitutes accommodation part 10 of culture plate 1. Recess 31 has a circular outer shape (outer edge part) in plan view.

The diameter of the outer shape of recess 31D and the diameter of the outer shape of through-hole 210a are equal to or substantially equal to each other. Note that the thickness relationship between recess 31D and flat surface part 30 is the same as the thickness relationship between recess 31 and flat surface part 30 in the above-described embodiment.

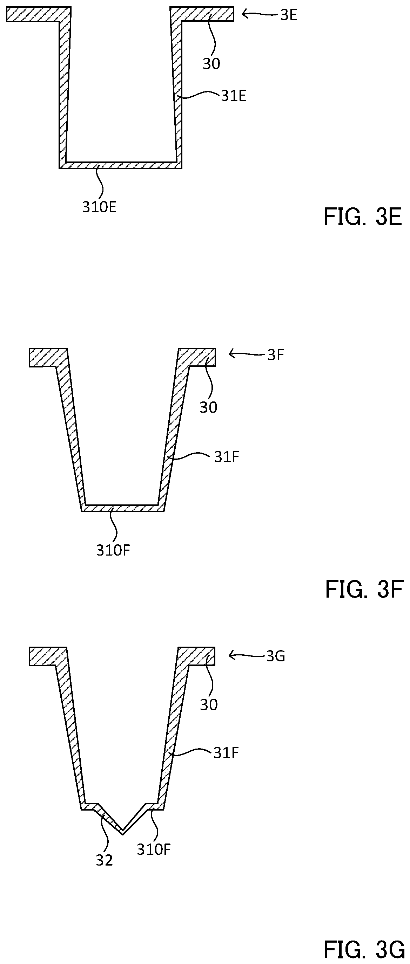

Next, gas-permeable sheet 3E according to modification example 5 illustrated in FIG. 3E includes recess 31E having a substantially rectangular cross-sectional shape. Recess 31E is disposed below through-hole 210a constituted by the inner space of cylindrical part 210 (see FIG. 2).

Recess 31E is constituted by a cylindrical part that is convex toward the lower side. Recess 31E closes the lower side opening part of through-hole 210a (see FIG. 2).

Recess 31E, together with through-hole 210a, constitutes accommodation part 10 of culture plate 1. Recess 31 has a circular outer shape (outer edge part) in plan view.

The diameter of the outer shape of recess 31E and the diameter of the outer shape of through-hole 210a are equal to or substantially equal to each other.

In the present example, the thickness of the side wall part of recess 31E decreases toward bottom part 310E. Further, in recess 31E, the thickness of bottom part 310E is the thinnest. The thickness relationship between recess 31E and flat surface part 30 is the same as the thickness relationship between recess 31 and flat surface part 30 in the above-described embodiment.

Next, gas-permeable sheet 3F according to modification example 6 illustrated in FIG. 3F includes recess 31F having a cross-sectional shape of a substantially inverted trapezoid. Recess 31F is disposed below through-hole 210a constituted by the inner space of cylindrical part 210 (see FIG. 2).

Recess 31F is constituted by an inverted conical frustum part that is convex toward the lower side. Recess 31F closes the lower side opening part of through-hole 210a (see FIG. 2).

Recess 31F, together with through-hole 210a, constitutes accommodation part 10 of culture plate 1. Recess 31 has a circular outer shape (outer edge part) in plan view.

The diameter of the outer shape of recess 31F and the diameter of the outer shape of through-hole 210a are equal to or substantially equal to each other.

In the present example, the thickness of the side wall part of recess 31F decreases toward bottom part 310F. Further, in recess 31F, the thickness of bottom part 310F is the thinnest. The thickness relationship between recess 31F and flat surface part 30 is the same as the thickness relationship between recess 31 and flat surface part 30 in the above-described embodiment.

Note that as in gas-permeable sheet 3G illustrated in FIG. 3G, second recess 32 (for example, a conical recess) that is convex toward the lower side may be provided at bottom part 310F of recess 31F.

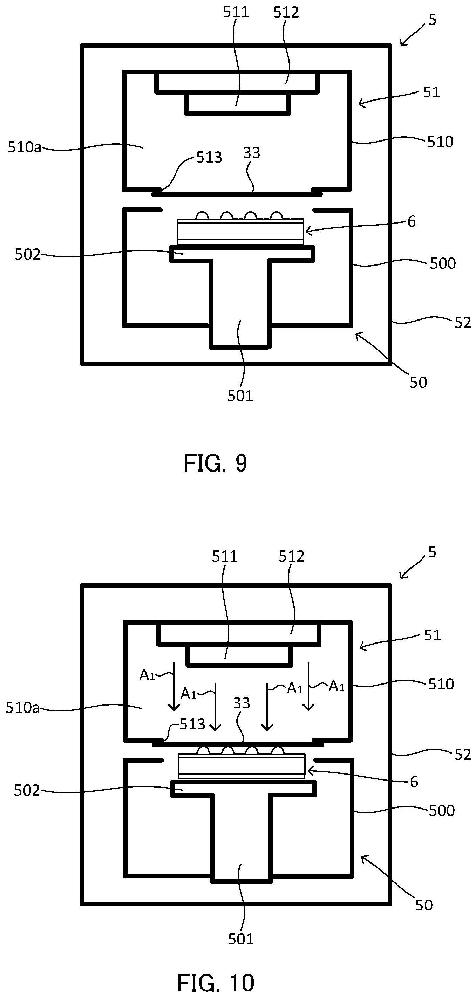

Next, a method for manufacturing culture plate 1 of the present embodiment will be described with reference to FIGS. 4 to 11. First, manufacturing apparatus 5 illustrated in FIGS. 9 and 10 is used in the manufacturing method of culture plate 1.

Here, the configuration of manufacturing apparatus 5 will be briefly described with reference to FIGS. 9 and 10.

Manufacturing apparatus 5 includes lower-side apparatus 50 and upper-side apparatus 51. Lower-side apparatus 50 and upper-side apparatus 51 are each fixed to fixed part 52.

Lower-side apparatus 50 includes lower-side housing 500, lifting apparatus 501, and mounting table 502.

Lower-side housing 500 is a box-shaped member. Lower-side housing 500 is a part that is fixed to fixed part 52.

Lifting apparatus 501 is accommodated in lower-side housing 500. Lifting apparatus 501 moves in the up-down direction under the control of a control part (not illustrated).

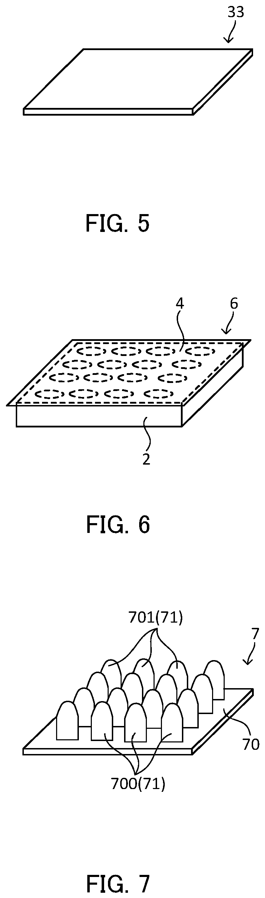

Mounting table 502 is fixed to the upper end part of lifting apparatus 501. Mounting table 502 is a table for mounting intermediate generation member 6 illustrated in FIG. 6. Mounting table 502 moves in the up-down direction together with lifting apparatus 501.

Upper-side apparatus 51 is provided above lower-side apparatus 50. Upper-side apparatus 51 includes upper-side housing 510, heater 511, blower 512, and sheet support part 513.

Upper-side housing 510 is a box-shaped member. Upper-side housing 510 is a part that is fixed to fixed part 52.

Heater 511 is accommodated in upper-side housing 510. Heater 511 heats inner space 510a of upper-side housing 510 under the control of a control part (not illustrated). Note that heater 511 may directly heat sheet material 33.

Blower 512 is accommodated in upper-side housing 510. Blower 512 blows compressed air toward sheet material 33 supported by sheet support part 513. Note that blower 512 may be omitted.

Sheet support part 513 is a part that supports sheet material 33. Sheet material 33 corresponds to an example of a planar sheet. Sheet material 33 is disposed above intermediate generation member 6 placed on mounting table 502 in a state of being supported by sheet support part 513. Sheet material 33, in a state of being supported by sheet support part 513, faces intermediate generation member 6 placed on mounting table 502 in the up-down direction.

The operation of manufacturing apparatus 5 will be described later. Further, the configuration of the manufacturing apparatus is not limited to the configuration of the above-described manufacturing apparatus 5. The manufacturing apparatus does not need to be divided into a lower-side apparatus and an upper-side apparatus. It suffices that the manufacturing apparatus includes a minimum configuration necessary to implement the manufacturing method of culture plate 1 described later at an appropriate position. A known apparatus can be used as the manufacturing apparatus. The manufacturing apparatus may be, for example, a vacuum forming machine (manufactured by Fu-se Vacuum Forming Ltd.) or a pressure forming machine (manufactured by Fu-se Vacuum Forming Ltd.).

Hereinafter, the steps implemented in a method for manufacturing culture plate 1 will be described. FIG. 4 is a flowchart illustrating steps performed in the method for manufacturing culture plate 1. Note that the following description assumes that main body part 2 of culture plate 1 has been produced in advance. Further, the order of steps illustrated in FIG. 4 may be changed as appropriate, provided that no technical contradictions arise.

In step S101 of FIG. 4, adhesive layer 4 is provided on the first surface of main body part 2. The first surface of main body part 2 corresponds to the lower surface in the use state of culture plate 1.

The step performed in step S101 is referred to also as the adhesive layer preparation step. In the adhesive layer preparation step, for example, sheet-like adhesive layer 4 that has been generated in advance is bonded to the first surface of main body part 2.

In step S101, intermediate generation member 6 illustrated in FIG. 6 is generated by bonding adhesive layer 4 to the first surface of main body part 2. Note that in FIG. 6, main body part 2 of intermediate generation member 6 is illustrated schematically. The configuration of main body part 2 is as described above.

Note that intermediate generation member 6 includes main body part 2 and adhesive layer 4 provided on the first surface of main body part 2. The configurations of main body part 2 and adhesive layer 4 are as described above.

In step S102 of FIG. 4, jig 7 is assembled to intermediate generation member 6. Step S102 corresponds to an example of the step of disposing the jig on the base material.

Now jig 7 will be described with reference to FIG. 7. Jig 7 includes base part 70 and a plurality of protrusion parts 71.

Base part 70 has a plate shape. Each protrusion part 71 protrudes upward from the first side surface (specifically, the upper surface) of base part 70. Each protrusion part 71 includes columnar column part 700 provided on the base end side (specifically, the lower side), and recess forming part 701 provided at the distal end (specifically, the upper end) of column part 700.

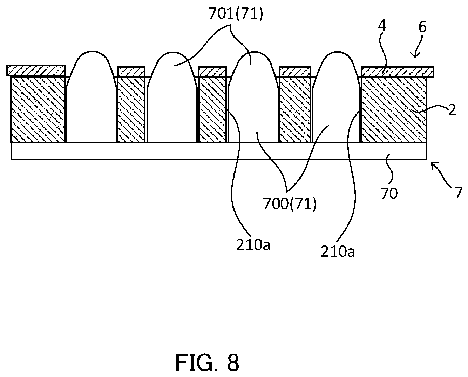

Column part 700 is a part that is inserted into through-hole 210a of main body part 2 in a state in which column part 700 is assembled to main body part 2 (see FIG. 8). The shape of column part 700 may be any shape that can be inserted into through-hole 210a of main body part 2.

Recess forming part 701 is a part that forms recess 31 of gas-permeable sheet 3. Recess forming part 701 protrudes upward from the upper surface of intermediate generation member 6 (that is, the upper end of through-hole 210a) in a state in which intermediate generation member 6 (in other words, main body part 2) is assembled (see FIG. 8).

Recess forming part 701 has a shape corresponding to recess 31. The shape of recess forming part 701 may be various shapes according to the shape of recess 31 of gas-permeable sheet 3.

Jig 7 having the above-described configuration is assembled to the intermediate generation member 6 from the lower surface of intermediate generation member 6 as illustrated in FIG. 8. **In** this state, recess forming part 701 of jig 7 protrudes upward from the upper surface of intermediate generation member 6 (in other words, main body part 2) (that is, the upper end of through-hole 210a). The lower surface of intermediate generation member 6 corresponds to the first surface of the base material. Further, the upper surface of intermediate generation member 6 corresponds to the second surface of the base material.

In step S103 of FIG. 4, intermediate generation member 6 (in other words, main body part 2) in which jig 7 is assembled is set (in other words, provided) in manufacturing apparatus 5 (see FIG. 9). Step S103 corresponds to an example of the step of providing a base material.

Further, step S103 also corresponds to an example of the step of providing, in a combined state, main body part 2 (base material) including a plurality of through-holes 201a and jig 7 including protrusion part 71 at a position corresponding to the plurality of through-holes 201a in holding body part 2.

Specifically, intermediate generation member 6 to which jig 7 is assembled is placed on mounting table 502 of manufacturing apparatus 5. In this state, intermediate generation member 6 is fixed to mounting table 502 by a fixing means (not illustrated).

In step S104 of FIG. 4, sheet material 33 is provided to manufacturing apparatus 5 (see FIG. 9). Sheet material 33 corresponds to an example of a gas-permeable sheet. Specifically, sheet material 33 is fixed to sheet support part 513 of manufacturing apparatus 5. Sheet material 33 is disposed on the upper surface side of intermediate generation member 6. The upper surface side of intermediate generation member 6 corresponds to an example of the first surface side of the base material. The step performed in step S104 corresponds to an example of the step of disposing a gas-permeable sheet.

In step S105 of FIG. 4, gas-permeable sheet 3 is generated and bonded to the upper surface of intermediate generation member 6. The process performed in step S105 corresponds to an example of the step of obtaining a gas-permeable sheet and the step of attaching the gas-permeable sheet. Further, the step performed in step S105 corresponds to an example of the step of forming a recess in the gas-permeable sheet and attaching the gas-permeable sheet to the base material.

That is, in step S105, a step of forming recess 31 in sheet material 33 (gas-permeable sheet) by bringing heated sheet material 33 (gas-permeable sheet) into pressure contact with protrusion part 71 (specifically, recess forming part 701) in a state in which protrusion part 71 (specifically, recess forming part 701) of jig 7 is protruded to the upper surface side (first surface side) of main body part 2 from through-hole 210a, and attaching sheet material 33 (gas-permeable sheet) to main body part 2 is performed.

Specifically, in step S105, the control part (not illustrated) of manufacturing apparatus 5 moves up lifting apparatus 501 in the state illustrated in FIG. 9. Note that the control part may control lifting apparatus 501 based on an instruction from the worker or may control lifting apparatus 501 based on a pre-installed program.

Under the control of the control part, lifting apparatus 501 moves to a position where recess forming part 701 of jig 7 comes into contact with or comes close to sheet material 33 supported by sheet support part 513.

Next, the control part heats inner space 510a of upper-side housing 510 with heater 511. In other words, heater 511 heats sheet material 33 supported by sheet support part 513. Note that heater 511 may start operating in a step prior to step S105.

Next, the control part drives blower 512 after heating sheet material 33 to a predetermined temperature. Under the control of the control part, blower 512 blows compressed air onto the upper surface of sheet material 33 supported by sheet support part 513 as indicated by arrow $A_1$ in FIG. 10.

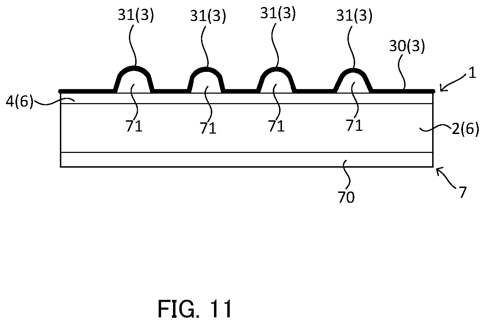

Then, sheet material 33 is deformed by being pressed against recess forming part 701 of jig 7. In a part of sheet material 33 that is pressed against recess forming part 701, recess 31 of gas-permeable sheet 3 is formed as illustrated in FIG. 11. Note that in step S105, the temperature for heating inner space 510a may be determined in relation to the glass transition temperature of the material constituting main body part 2 of intermediate generation member 6 and the glass transition temperature of the material constituting sheet material 33 (in other words, gas-permeable sheet 3). The temperature for heating inner space 510a is preferably a temperature at which sheet material 33 deforms but main body part 2 does not deform.

As described above, in the present embodiment, when forming recess 31 in gas-permeable sheet 3, blower 512 blows compressed air onto the upper surface of sheet material 33. This configuration can deform gas-permeable sheet 3 with high accuracy in accordance with the shape of recess forming part 701 even in a case where recess formation part 701 of jig 7 has a fine shape. Note that a configuration in which compressed air is not blown onto sheet material 33 can also be selected depending on the shape of recess forming part 701. In this case, gas-permeable sheet 3 may be formed by pressing sheet material 33 into recess forming part 701 by a so-called atmospheric pressure release.

Further, in sheet material 33, the part that is pressed against adhesive layer 4 of intermediate generation member 6 is bonded to intermediate generation member 6 via adhesive layer 4, as illustrated in FIG. 11. The part of sheet material 33 that is bonded to adhesive layer 4 corresponds to flat surface part 30 of gas-permeable sheet 3. In this manner, gas-permeable sheet 3 is generated.

After step S105, intermediate generation member 6 to which gas-permeable sheet 3 is attached is cooled. Then, by removing the extra gas-permeable sheet 3 that protrudes from the outer edge of intermediate generation member 6, culture plate 1 as illustrated in FIG. 11 is completed.

Note that in FIG. 11, culture plate 1 is illustrated schematically. Further, FIG. 11 also illustrates jig 7. Jig 7 may be removed from culture plate 1 at an appropriate timing. The specific configuration of culture plate 1 is as described above. Further, after step S105, a treatment such as antibacterial treatment may be appropriately performed.

As described above, in the present embodiment, the step of obtaining a gas-permeable sheet and the step of attaching the gas-permeable sheet are performed simultaneously in step S105.

Note that the step of obtaining the gas-permeable sheet and the step of attaching the gas-permeable sheet may be performed at different timings. That is, after the step of obtaining the gas-permeable sheet is performed, the step of bonding the gas-permeable sheet may be performed at an appropriate timing.

Finally, a method for culturing a spheroid using culture plate 1 of the present embodiment described above will be briefly described. The worker puts a culture solution and target cells into accommodation part 10 of culture plate 1. The target cell may be, for example, a cell collected from a human. The culture solution may be appropriately selected according to the target cell. The target cell is accommodated in the lower side region of accommodation part 10 (that is, recess 31 of gas-permeable sheet 3).

Next, the worker accommodates culture plate 1 in a culture space of a culture apparatus (not illustrated) such as an incubator. The environmental conditions of the culture space are set to an environment suitable for culturing the target cells. Subsequently, after a predetermined time has elapsed, cells are cultured in accommodation part 10 of culture plate 1.

In culture plate 1 of the present embodiment, the lower side region of accommodation part 10 is constituted by recess 31 of gas-permeable sheet 3. Gas-permeable sheet 3 is excellent in oxygen permeability, and thus, a large amount of oxygen is supplied to the target cells accommodated in the lower region of accommodation part 10. In this manner, the culture of the target cells is promoted.

Further, the cultured cells are likely to gather in the lower side region of accommodation part 10 due to its shape recessed downward. For this reason, a spheroid (cell aggregate) of the target cell is easily formed in the lower region of accommodation part 10. Thus, culture plate 1 of the present embodiment can be suitably used for culturing spheroids (cell aggregates) of target cells.

Actions and Effects of the Present Embodiment

As described above, according to culture plate 1 of the present embodiment, a large amount of oxygen can be supplied to the target cells accommodated in the lower side region of accommodation part 10, thereby promoting the culture of the target cells. Thus, according to culture plate 1 of the present embodiment, it is possible to provide a culture plate with a high culture function. The actions and effects that can be achieved with culture plate 1 of the present embodiment are as described above.

Supplementary Notes

In a case where culture plate 1 of the above-described embodiment is implemented, a gas blocking sheet (not illustrated) that blocks the permeation of a gas (specifically, oxygen) may be provided on the lower surface of gas-permeable sheet 3. Culture plate 1 having such a configuration is switchable between a state in which air is not supplied to accommodation part 10 via recess 31 of gas-permeable sheet 3 (hereinafter referred to also as "first state of culture plate 1") and a state in which air is supplied to accommodation part 10 via recess 31 of gas-permeable sheet 3 (hereinafter referred to also as "second state of culture plate 1").

The first state of culture plate 1 corresponds to the state in which a gas blocking sheet is provided on the lower surface of gas-permeable sheet 3. The second state of culture plate 1 corresponds to the state in which the gas blocking sheet is removed from the lower surface of gas-permeable sheet 3.

Further, the shapes of the recesses of the gas-permeable sheet may be different from each other in properties (for example, diameter, depth, or shape). For example, in one culture plate, the gas-permeable sheet may include at least two types of recesses selected from recesses 31 to 31G illustrated in FIGS. 2 and 3A to 3G described above.

The manufacturing method of culture plate 1 according to the above-described embodiment uses a so-called pressure air type manufacturing apparatus 5 that blows compressed air onto sheet material 33. Note that the manufacturing apparatus is not limited to the pressure air type manufacturing apparatus 5.

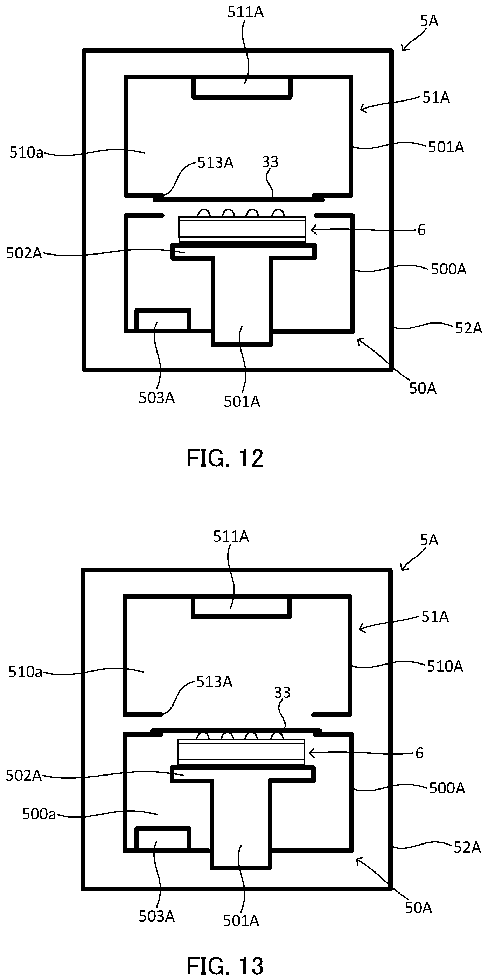

FIGS. 12 and 13 are schematic views illustrating a modification example of the manufacturing apparatus. Manufacturing apparatus 5A illustrated in FIGS. 12 and 13 is a so-called vacuum-type manufacturing apparatus. Hereinafter, the configuration of manufacturing apparatus 5A will be briefly described.

Manufacturing apparatus 5A includes lower-side apparatus 50A and upper-side apparatus 51A. Lower-side apparatus 50A and upper-side apparatus 51A are each fixed to fixed part 52A.

Lower-side apparatus 50A includes lower-side housing 500A, lifting apparatus 501A, and mounting table 502A. The configurations of lower-side housing 500A, lifting apparatus 501A, and mounting table 502A are the same as those of lower-side housing 500, lifting apparatus 501, and mounting table 502 of manufacturing apparatus 5 in the above-described embodiment.

In particular, in the present example, lower-side apparatus 50A includes vacuum apparatus 503A that makes inner space 500a of lower-side housing 500 into a vacuum state.

Upper-side apparatus 51A is provided above lower-side apparatus 50A. Upper-side apparatus 51A includes upper-side housing 510A, heater 511A, and sheet support part 513A. The configurations of upper-side housing 510A, heater 511A, and sheet support part 513A are substantially the same as the configurations of upper-side housing 510, heater 511, and sheet support part 513 in manufacturing apparatus 5 in the above-described embodiment. Note that upper-side apparatus 51A in the present example does not include a blower.

Hereinafter, the operation of manufacturing apparatus 5A will be briefly described. In the state of manufacturing apparatus 5A illustrated in FIG. 12, sheet material 33 is supported by sheet support part 513A. Further, intermediate generation member 6 is placed on mounting table 502A. The height of mounting table 502A is adjusted to an appropriate height by the movement of lifting apparatus 501A.

In the state illustrated in FIG. 12, the control part (not illustrated) of manufacturing apparatus 5A drives heater 511A. Then, sheet material 33 supported by sheet support part 513A is heated by heater 511A. Next, the control part of manufacturing apparatus 5A drives vacuum apparatus 503A.

Then, inner space 500a of lower-side housing 500 is depressurized toward a vacuum state. Then, sheet material 33 supported by sheet support part 513A is drawn to the inner space 500a side.

As a result, sheet material 33 is deformed by being pressed against recess forming part 701 of jig 7 (see FIG. 8). In a part of sheet material 33 that is pressed against recess forming part 701, recess 31 of gas-permeable sheet 3 is formed as illustrated in FIG. 11.

Further, in sheet material 33, the part that is pressed against adhesive layer 4 of intermediate generation member 6 is bonded to intermediate generation member 6 via adhesive layer 4, as illustrated in FIG. 11. The part of sheet material 33 that is bonded to adhesive layer 4 corresponds to flat surface part 30 of gas-permeable sheet 3. In this manner, gas-permeable sheet 3 is generated.

Subsequently, intermediate generation member 6 to which gas-permeable sheet 3 is attached is cooled. Then, by removing the extra gas-permeable sheet 3 that protrudes from the outer edge of intermediate generation member 6, culture plate 1 as illustrated in FIG. 11 is completed.

The disclosure of Japanese Patent Application Nos. 2022-204758 and 2022-204761, filed on December 21, 2022, including the specification, drawings and abstract, is incorporated herein by reference in its entirety.

Industrial Applicability

The culture plate according to the present invention can be applied to the culture of various cells.

Reference Signs List

    1 Culture plate
    10 Accommodation part
    2 Main body part
    20 Frame part
    200 Lower frame part
    200a Notch part
    201 Upper frame part
    21 Main body-side accommodation part
    210 Cylindrical part
    210a Through-hole
    211 Bottom plate part
    3, 3A, 3B, 3C, 3D, 3E, 3F, 3G Gas-permeable sheet
    30 flat surface part
    31, 31A, 31B, 31C, 31D, 31E, 31F, 31G Recess
    310, 310E, 310F Bottom part
    311 Curved surface part
    32 Second recess
    33 Sheet material
    4 Adhesive layer
    5, 5A Manufacturing apparatus
    50 Lower-side apparatus
    500 Lower-side housing
    500a Inner space
    501 Lifting apparatus
    502 Mounting table
    503A Vacuum apparatus
    51 Upper-side apparatus
    510 Upper-side housing
    510a Inner space
    511 Heater
    512 Blower
    513 Sheet support part
    52 Fixed part
    6 Intermediate generation member
    7 Jig
    70 Base part
    71 Protrusion part
    700 Column part
    701 Recess forming part

**Claims**

1. A culture plate comprising:

    a plurality of accommodation parts for culturing cells;
    a base material including a plurality of through-holes; and
    a gas-permeable sheet, the gas-permeable sheet including:

        a flat surface part attached to a lower surface of the base material, and
        a plurality of recesses disposed below the through-holes so as to close the through-holes and form the accommodation parts.

2. The culture plate according to claim 1, wherein a thickness of the recess is smaller than a thickness of the flat surface part.

3. The culture plate according to claim 1, wherein in the recess, a bottom part has a thickness smaller than a thickness of a peripheral part.

4. The culture plate according to claim 3, wherein the thickness of the recess decreases toward the bottom part.

5. The culture plate according to claim 2, wherein a maximum thickness $T_{1max}$ of the bottom part and an average thickness $T_2$ of the flat surface part satisfy a relationship of

$$0.5T_2 \leq T_{1max} \leq 0.9T_2.$$

6. The culture plate according to claim 2, wherein a thickness $T_{1mid}$ of a central part of the recess in an up-down direction and an average thickness $T_2$ of the flat surface part satisfy a relationship of $0.65T_2 \leq T_{1mid} \leq 0.98T_2$.

7. The culture plate according to claim 1, wherein an oxygen permeability coefficient of the gas-permeable sheet is 200 to 60,000 $cm^3 \times mm/(m^2 \times 24\,hr \times atm)$.

8. The culture plate according to claim 1, wherein the base material and the gas-permeable sheet are constituted by thermoplastic resins different from each other.

9. The culture plate according to claim 1, wherein a glass transition temperature of a thermoplastic resin constituting the base material is higher by 40°C or more than a glass transition temperature of a thermoplastic resin constituting the gas-permeable sheet.

10. The culture plate according to claim 1, wherein the gas-permeable sheet includes a polymer having a structural unit derived from 4-methyl-1-pentene.

11. The culture plate according to claim 1, wherein a bottom surface of the recess includes a curved surface part having a curvature radius R1 that satisfies $0.5 \text{ mm} \leq R1 \leq 2 \text{ mm}$.

12. The culture plate according to claim 1, wherein the base material includes a lower peripheral wall part at a lower part, the lower peripheral wall part having a height with which a deepest part of the recess does not come into contact with a mounting surface.

13. The culture plate according to claim 12, wherein the base material includes an upper peripheral wall part at an upper part, the upper peripheral wall part being fitted to the lower peripheral wall part of another culture plate.

14. The culture plate according to claim 12, wherein the lower peripheral wall part includes a cutout part.

15. The culture plate according to claim 1, wherein the base material is constituted by polystyrene or polyolefin.

16. The culture plate according to claim 1, wherein an instantaneous value of a Shore D hardness of the base material is 60 to 90.

17. A method for manufacturing a culture plate, the method comprising:

   providing a base material including a plurality of through-holes;
   obtaining a gas-permeable sheet by deforming a planar sheet and forming a plurality of recesses at positions corresponding to the through-holes; and
   bonding the gas-permeable sheet to the base material in a state in which the plurality of recesses are disposed at positions of the plurality of through-holes.

18. A method for manufacturing a culture plate, the method comprising:

   providing a base material and a jig in a combined state, the base material including a plurality of through-holes, the jig including a protrusion at a position corresponding to the plurality of through-holes;
   disposing a gas-permeable sheet on a first surface side of the base material; and

forming a recess in the gas-permeable sheet by pressing against the protrusion part the gas-permeable sheet that is heated, in a state where the protrusion part protrudes from the through-hole to the first surface side, and attaching the gas-permeable sheet to the base material.

19. The method for manufacturing the culture plate according to claim 18, further comprising providing an adhesive layer on the first surface of the base material before the providing of the jig.

20. The method for manufacturing the culture plate according to claim 18, wherein compressed air is blown onto the gas-permeable sheet to press the gas-permeable sheet against the jig and form a recess in the gas-permeable sheet, and bond the gas-permeable sheet to the base material.

21. The method for manufacturing the culture plate according to claim 18, wherein a pressure in a space in which the base material is disposed is reduced to press the gas-permeable sheet against the jig and form a recess in the gas-permeable sheet, and bond the gas-permeable sheet to the base material.

22. A method for culturing a spheroid by using the culture plate according to claim 1.

FIG. 1

FIG. 2

FIG. 3A

FIG. 3B

FIG. 3C

FIG. 3D

FIG. 3E

FIG. 3F

FIG. 3G

START

PROVIDE ADHESIVE LAYER AT MAIN BODY PART — S101

ATTACH JIG TO INTERMEDIATE GENERATION MEMBER — S102

SET INTERMEDIATE GENERATION MEMBER AT MANUFACTURING APPARATUS — S103

DISPOSE GAS-PERMEABLE SHEET — S104

GENERATE AND BOND GAS-PERMEABLE SHEET — S105

END

FIG. 4

33

FIG. 5

4    6

2

FIG. 6

701(71)

7

70

700(71)

FIG. 7

FIG. 8

FIG. 9

FIG. 10

31(3)  31(3)  31(3)  31(3)

30(3)

1

4(6)

71  71  71  71

2(6)

70

7

## FIG. 11

FIG. 12

FIG. 13

## INTERNATIONAL SEARCH REPORT

| International application No. |
| --- |
| **PCT/JP2023/032030** |

| A. CLASSIFICATION OF SUBJECT MATTER |
| --- |
| *C12M 1/00*(2006.01)i; *C12M 3/04*(2006.01)i; *C12N 5/071*(2010.01)i<br>FI: C12M1/00 D; C12N5/071; C12M3/04 |
| According to International Patent Classification (IPC) or to both national classification and IPC |

| B. FIELDS SEARCHED |
| --- |
| Minimum documentation searched (classification system followed by classification symbols)<br>C12M1/00; C12M3/04; C12N5/071 |
| Documentation searched other than minimum documentation to the extent that such documents are included in the fields searched<br>Published examined utility model applications of Japan 1922-1996<br>Published unexamined utility model applications of Japan 1971-2023<br>Registered utility model specifications of Japan 1996-2023<br>Published registered utility model applications of Japan 1994-2023 |
| Electronic data base consulted during the international search (name of data base and, where practicable, search terms used)<br>CAplus/MEDLINE/EMBASE/BIOSIS (STN) |

| C. DOCUMENTS CONSIDERED TO BE RELEVANT | | |
| --- | --- | --- |
| Category* | Citation of document, with indication, where appropriate, of the relevant passages | Relevant to claim No. |
| X | WO 2022/138101 A1 (MITSUI CHEMICALS, INC.) 30 June 2022 (2022-06-30)<br>abstract, claims, paragraphs [0005]-[0007], [0022]-[0044], [0068]-[0074], [0111]-[0114] | 1, 7-10, 15-22 |
| Y | | 2-6, 11-14 |
| X | WO 2020/256079 A1 (MITSUI CHEMICALS, INC.) 24 December 2020 (2020-12-24)<br>abstract, claims, paragraphs [0011]-[0013], [0037]-[0041], [0049]-[0067], [0082]-[0089], [00112]-[0144] | 1, 7-10, 15-22 |
| Y | | 2-6, 11-14 |
| Y | JP 2017-532971 A (CORNING INCORPORATED) 09 November 2017 (2017-11-09)<br>abstract, paragraphs [0002], [0026], [0039], [0040], fig. 2A | 1-22 |
| Y | WO 2008/060521 A1 (ACME BIOSYSTEMS, LLC) 22 May 2008 (2008-05-22)<br>abstract, claims, fig. 6A-6C | 1-22 |
| A | JP 2022-99831 A (MITSUI CHEMICALS, INC.) 05 July 2022 (2022-07-05)<br>abstract, claims | 1-22 |

☑ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **13 November 2023** | **21 November 2023** |

| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br>Telephone No. |
| --- | --- |

Form PCT/ISA/210 (second sheet) (January 2015)

**INTERNATIONAL SEARCH REPORT**

International application No.

**PCT/JP2023/032030**

**C.    DOCUMENTS CONSIDERED TO BE RELEVANT**

| Category* | Citation of document, with indication, where appropriate, of the relevant passages | Relevant to claim No. |
|---|---|---|
| A | JP 61-108372 A (BECTON, DICKINSON AND COMPANY) 27 May 1986 (1986-05-27) claims, fig. 1-5 | 1-22 |

Form PCT/ISA/210 (second sheet) (January 2015)

## INTERNATIONAL SEARCH REPORT
### Information on patent family members

International application No.

**PCT/JP2023/032030**

| Patent document cited in search report | | | Publication date (day/month/year) | Patent family member(s) | Publication date (day/month/year) |
|---|---|---|---|---|---|
| WO | 2022/138101 | A1 | 30 June 2022 | EP 4269558 A1 abstract, claims, paragraphs [0005]-[0007], [0024]-[0048], [0075]-[0081], [0137]-[0140] CN 116583595 A | |
| WO | 2020/256079 | A1 | 24 December 2020 | US 2021/0309954 A1 abstract, claims, paragraphs [0019]-[0021], [0058]-[0065], [0074]-[0102], [0126]-[0136], [0215]-[0247] EP 3839034 A1 | |
| JP | 2017-532971 | A | 09 November 2017 | US 2017/0226455 A1 abstract, paragraphs [0002], [0026], [0048], [0049], fig. 2A US 2022/0081661 A1 WO 2016/069930 A1 EP 3212761 A1 | |
| WO | 2008/060521 | A1 | 22 May 2008 | US 2010/0055790 A1 abstract, claims, fig. 6A-6C EP 2126037 A1 | |
| JP | 2022-99831 | A | 05 July 2022 | (Family: none) | |
| JP | 61-108372 | A | 27 May 1986 | US 4657867 A claims, fig. 1-5 EP 183973 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022149254 A
- JP 2022204758 A
- JP 2022204761 A